# EUROPEAN PATENT SPECIFICATION

(11) **EP 4 151 224 B1**
(45) Date of publication and mention of the grant of the patent: **31.12.2025**
(21) Application number: 21803905.5
(22) Date of filing: 14.05.2021
(51) Int. Cl.: A61K 35/15, A61K 35/35, A61K 39/00, A61P 35/00, A61K 39/39, C12N 5/0775, C12N 5/0784, A61K 40/24, A61K 40/42, A61K 40/19

(54) **COMPOSITION FOR ENHANCING IMMUNE RESPONSE BY USING ACTIVATION FUNCTION OF DENDRITIC CELLS OF STROMAL VASCULAR FRACTIONS ISOLATED FROM ADIPOSE TISSUES**
ZUSAMMENSETZUNG ZUR VERSTÄRKUNG DER IMMUNREAKTION DURCH VERWENDUNG DER AKTIVIERUNGSFUNKTION DENDRITISCHER ZELLEN AUS STROMAGEFÄSSFRAKTIONEN AUS FETTGEWEBE
COMPOSITION POUR AMÉLIORER LA RÉPONSE IMMUNE PAR L'UTILISATION D'UNE FONCTION D'ACTIVATION DE CELLULES DENDRITIQUES DE FRACTIONS VASCULAIRES STROMALES ISOLÉES À PARTIR DE TISSUS ADIPEUX

(30) Priority: 15.05.2020 KR 20200058661
(43) Date of publication of application: 22.03.2023
(73) Proprietor: Seoul National University R & DB Foundation, Seoul 08826 (KR); Korea University Research and Business Foundation, Seoul 02841 (KR)
(72) Inventor: CHO, Nam Hyuk, Seoul 01792 (KR); LEE, Jae Won, Seoul 02863 (KR); JANG, Na Yoon, Seoul 03081 (KR); KIM, Young Keun, Seoul 06009 (KR); PARK, Bum Chul, Seoul 04700 (KR)
(74) Representative: V.O.
(86) International application number: PCT/KR2021/006068
(87) International publication number: WO 2021/230704

(56) References cited:
- KR-A- 20180 042 284
- US-A1- 2012 164 113
- MUELLER SCOTT N. ET AL: "Stromal cell contributions to the homeostasis and functionality of the immune system", NATURE REVIEWS IMMUNOLOGY, vol. 9, no. 9, 31 July 2009 (2009-07-31), London, pages 618 - 629, XP093124330, ISSN: 1474-1733, Retrieved from the Internet <URL:http://www.nature.com/articles/nri2588> DOI: 10.1038/nri2588
- BI HONGSEN ET AL: "Stromal vascular fraction promotes migration of fibroblasts and angiogenesis through regulation of extracellular matrix in the skin wound healing process", STEM CELL RESEARCH & THERAPY, vol. 10, no. 1, 17 October 2019 (2019-10-17), London, UK, XP093124348, ISSN: 1757-6512, Retrieved from the Internet <URL:http://link.springer.com/article/10.1186/s13287-019-1415-6/fulltext.html> DOI: 10.1186/s13287-019-1415-6
- WCULEK STEFANIE K.; CUETO FRANCISCO J.; MUJAL ADRIANA M.; MELERO IGNACIO; KRUMMEL MATTHEW F.; SANCHO DAVID: "Dendritic cells in cancer immunology and immunotherapy", NATURE REVIEWS IMMUNOLOGY, NATURE PUBLISHING GROUP UK, LONDON, vol. 20, no. 1, 29 August 2019 (2019-08-29), London, pages 7 - 24, XP036976389, ISSN: 1474-1733, DOI: 10.1038/s41577-019-0210-z
- BOWLES ANNIE C., WISE RACHEL M., GERSTEIN BRITTANY Y., THOMAS ROBERT C., OGELMAN ROBERTO, FEBBO ISABELLA, BUNNELL BRUCE A.: "Immunomodulatory Effects of Adipose Stromal Vascular Fraction Cells Promote Alternative Activation Macrophages to Repair Tissue Damage", STEM CELLS, vol. 35, no. 10, 1 October 2017 (2017-10-01), pages 2198 - 2207, XP055869666, ISSN: 1066-5099, DOI: 10.1002/stem.2689
- PER ANDERSON, ELENA GONZALEZ-REY, FRANCISCO O’VALLE, FRANCISCO MARTIN, F. JAVIER OLIVER, MARIO DELGADO: "Allogeneic Adipose-Derived Mesenchymal Stromal Cells Ameliorate Experimental Autoimmune Encephalomyelitis by Regulating Self-Reactive T Cell Responses and Dendritic Cell Function", STEM CELLS INTERNATIONAL, HINDAWI PUBLISHING CORPORATION, US, vol. 2017, 1 January 2017 (2017-01-01), US , pages 1 - 15, XP055663479, ISSN: 1687-966X, DOI: 10.1155/2017/2389753

## Description

### Technical Field

The present disclosure relates to a composition for enhancing an immune response using the activation function of dendritic cells of the stromal vascular fraction isolated from adipose tissue. More particularly, the present disclosure relates to a composition for enhancing an immune response for anti-tumor use.

### Background Art

Dendritic cells strongly induce antigen-specific T cell responses in adaptive immunity. Promotion of dendritic cells in T cell responses is particularly important in anti-tumor immune responses. Dendritic cells capture tumor antigens that appear to be characteristic of tumor cells and transport tumor antigens to the draining lymph node. In lymph nodes, dendritic cells present their tumor antigens as cytotoxic T lymphocytes through cross presentation. Based on their important role in anti-tumor efficacy and the safety of clinical application, dendritic cells are used in cancer immunotherapy. Although the first clinical trial was in the 1990s, the treatment response rate was less than 15% for 20 years. To improve the low response rate, dendritic cell therapy must overcome several obstacles. First, it is important to create an opportunity for dendritic cells to migrate to lymph nodes and meet with T cells to initiate an adaptive immune response. Several papers reported that only less than 15% of dendritic cells could migrate to lymph nodes, and most dendritic cells remain in the injected area. Moreover, the tumor microenvironment inhibits the anti-tumor immune response of lymph nodes near the tumor as well as the tumor site, which limits the ability of dendritic cell-based immunotherapy. Therefore, in order to increase antigen-specific immune responses and fully exploit the potential of dendritic cell-based treatments, it is essential to use the dendritic cells remaining in the injected area and avoid the effects of the tumor microenvironment.

Dendritic cells meet T cells at specialized locations called "specialized compartments of lymphoid organs" for proper T cell activation. This organ is composed of different CD45-non-hematopoietic stromal cells, including fibroblastic reticular cells (FRC) and endothelial cells. In general, stromal cells can be classified by confirmation of expression of fibroblast markers podoplanin (PDPN, gp38) and endothelial cell marker CD31 (platelet endothelial cell adhesion molecule-1, PECAM-1). PDPN+ CD31-FRC provides structural support by constructing a conduit system that delivers soluble antigens and by forming a three-dimensional cellular network through which immune cells can migrate. The chemokines CCL19 and CCL21 produced by FRC bring in CCR7+ T cells and dendritic cells. As a result, the interaction between T cells and dendritic cells increases, the maturation of dendritic cells is promoted, and antigen presentation is made by dendritic cells. In addition, FRC regulates the homeostasis of T cells and dendritic cells through the secretion of interleukin (IL)-7 and IL-15. Additionally, PDPN expressed in FRC promotes dendritic cell motility. Recently, IL-6 secreted from FRC has led to improved survival and metabolism of CD8+ T cells through chromatin remodeling and to produce tissue-resident memory T cells. PDPN CD31+ lymphatic endothelial cells (LECs) constitute influent and outflow lymphatic vessels that coordinate the migration of leukocytes and lymph and regulate the differentiation of T cells. PDPN-CD31+ blood endothelial cells (BEC) constitute high endothelial venules (HEV) and blood vessels. BEC is involved in blood cell leakage of lymphocytes in the bloodstream. Therefore, since stromal cells are structurally and functionally helpful, dendritic cells in dendritic cell-based immunotherapy need the help of stromal cells that can induce a complete anti-tumor T-cell response. However, since stromal cells only exist in less than 5% of lymph nodes, it is virtually impossible to isolate these stromal cells and use the separated stromal cells directly for therapeutics.

Müller et al. (Nature Reviews Immunology, 9 (9), 2009, 618-629) discloses that subsets of stromal cells from secondary lymphoid organs, namely fibroblastic reticular cells (FRCs), interact with antigen present cells, in particular dendritic cells and that these interactions and chemokines produced by both cell types help induce immune responses.

The present disclosure discloses that the stromal vascular fraction (SVF) isolated from adipose tissue present in a considerable amount in the body can improve the induction of anti-tumor T cell response by dendritic cells, similar to stromal cells in lymph nodes.

### Disclosure

### Technical Problem

An objective of the present disclosure is to provide a composition for enhancing immune response using the activating function of dendritic cells of the stromal vascular fraction isolated from adipose tissue.

Other objectives and detailed objectives will be understood from the description given below.

### Technical Solution

As confirmed in the following embodiment, the inventors confirmed that stromal vascular fraction (SVF) separated and mixed visceral adipose tissue and subcutaneous adipose tissue and isolated from the mixture is composed of cells similar to lymph node stromal cells in phenotype or function and confirmed that SVF spheroids obtained by three-dimensional incubation of the SVF may form blood vessels that become moving channels for immune cells. It was confirmed that SVF spheroids highly expressed various factors (Spp1, Ccl8, Cxcl5, Postn, Cxcl16, Ccl17, etc.) such as chemokines that bring in and activate immune cells, and also highly expressed osteopontin (Spp1), a factor known to regulate the maturation and survival of dendritic cells. In addition, it was confirmed that SVF spheroids attract immune cells including dendritic cells in an in vitro test and enhance the interaction between dendritic cells and T cells when transplanted in vivo together with dendritic cells in an in vivo test. Furthermore, the present inventors found that when SVF two-dimensional culture is co-cultured in vitro with dendritic cells or transplanted in vivo with dendritic cells loaded with a specific antigen (OVA), SVF two-dimensional culture promotes the activation of dendritic cells and improves the survival rate of dendritic cells, thereby effectively activating T cells and enhancing the response of antigen-specific T cells. When SVF spheroids are injected together with dendritic cells loaded with the antigen (OAV) into mice transplanted with B16 melanoma cells (B16-OVA) expressing a specific antigen (OVA), it was confirmed that tumor growth was inhibited, and the survival rate of experimental animals was also improved.

The present disclosure is provided based on the above experimental results, and in one aspect, the present disclosure can be identified as a composition for enhancing immune response, containing a stromal vascular fraction isolated from adipose tissue, especially dendritic cells loaded with spheroids and antigens of the stromal vascular fraction as active ingredients. Herein, the immune response refers to an adaptive immune response, preferably a cell-mediated immune response, and more preferably, an immune response caused by a T cell.

In the present disclosure, the stromal vascular fraction isolated from adipose tissue may be used in a two-dimensional culture form or a three-dimensional culture spheroid form, but preferably in a spheroid form.

When the antigen loaded on dendritic cells is a virus-derived antigen, the composition for enhancing immunity of the present disclosure can be identified as an anti-viral composition for enhancing immunity for treatment or prevention of viral infection when the antigen loaded on dendritic cells is an antigen derived from pathogenic microorganisms such as bacteria, the composition can be identified as an anti-microbial composition for enhancing immunity for treatment or prevention of infections caused by such microorganisms, and when the antigen loaded on the dendritic cells is a tumor cell-derived antigen, the composition can be identified as an anti-tumor immune-enhancing composition for tumor treatment or prevention.

The composition for enhancing immunity of the present disclosure may be used for treating or preventing microbial infection such as arbitrary viral infection or arbitrary bacterial infection, and preferably may be used as anti-tumor application for treating or preventing an arbitrary tumor.

In the present disclosure, the dendritic cells may be autologous dendritic cells obtained from the subject to which the composition for enhancing immunity of the present disclosure is administered or may be allogeneic dendritic cells obtained from another subject or heterogeneous dendritic cells obtained from a heterogeneous subject. Dendritic cells obtained from heterogeneous subjects are obtained from mammals such as mice, rats, rabbits, monkeys, pigs, horses, cattle, sheep, antelopes, dogs, cats, etc., when the subject to which the composition for enhancing immunity of the present disclosure is administered is a human. These dendritic cells may be derived from bone marrow, peripheral blood, or umbilical cord blood.

In the present disclosure, the dendritic cells may be immature dendritic cells or mature dendritic cells, preferably mature dendritic cells. Compared to immature dendritic cells, mature dendritic cells express high concentrations of MHC type II molecules, T-cell auxiliary stimulating factors CD80, CD86, CD83, and immune-stimulating factors, such as cytokines IL-12, IL-10, and TNF, on the cell surface. Morphologically, immature dendritic cells are circular with smooth surfaces, but mature dendritic cells have rough surfaces and multiple pseudopodia. Techniques for maturation of immature dendritic cells are known in the art, and as such methods, for example, a method of culturing undifferentiated progenitor cells such as CD34+ hematopoietic cells, which can be isolated from bone marrow, peripheral blood, and umbilical cord blood and differentiated into microphages and dendritic cells, together with dendritic cell maturation factors such as IFN-α (European Patent No. 922,758), a method of culturing together with dendritic cell maturation factors such as GM-CSF, TNF-α, IL-3 (European Patent No. 663,930), a method of culturing with a mixture of hematopoietic growth factors and cytokines (WO 95/28479), a method of culturing with lipopolysaccharide (LPS) (Am. J. Physiol. Cell Physiol., 2011, 300, C1205-C1214; Annu. Rev. Immunol., 2003, 21, 335-376) and the like can be exemplified. All documents cited herein are considered as part of the present specification, including the above documents.

In the present disclosure, the stromal vascular fraction (SVF) isolated from adipose tissue may be obtained by being isolated from adipose tissue by enzymatic isolation methods and mechanical isolation methods.

In the enzymatic isolation method, first, adipose tissue or lipoaspirate is washed two to three times with an aqueous salt solution such as PBS, lactated Ringer's solution (LBS), Hank's balanced salt solution (HBSS), etc. After washing, an enzyme that decomposes extracellular matrix (ECM), etc., is treated in the washed adipose tissue or lipoaspirate. This enzymatic treatment is usually performed while stirring at 37°C for 30 minutes to 2 hours. After the enzymatic treatment, centrifugation is performed. Adipose tissue or lipoaspirate enzymatically treated by centrifugation is divided into (i) adipose tissue or oil layer, (ii) aqueous solution layer, and (ii) pellet layer, the remaining layer is discarded and the pellet layer is obtained. This pellet contains the stromal vascular fraction.

In this enzymatic isolation method, as the enzyme that can be used for the purpose of decomposing the extracellular matrix, etc., a first type of collagenase or a second type of collagenase isolated from Clostridium histolyticum, dispase, a neutral protease isolated from P. polymyxa, and thermolysin isolated from G. stearothermophilus or B. thermoproteolyticus may be used. Also, enzyme mixtures commercially available for this use may also be used, such as CIzyme^{™} AS (Vitacyte LLC, Indianapolis, Indiana), a mixture of first and second types of collagenase and dispase enzymes, or Liberase^{™} Research Grade (Roche Diagnostic, Based, Switzerland), which is a mixture of enzymes of the first and second types of collagenase and thermolysin.

In addition to the collagenase and the like, the enzyme may be used to improve the efficiency of cell isolation by decomposing chromosomes flowed from the dead cells when nucleic acid decomposers, such as DNA decomposition enzymes isolate cells.

The mechanical isolation method is performed by washing and centrifuging as in the above enzymatic isolation method to recover the pellet. In order to facilitate the recovery of the pellet before centrifugation, shaking/ vibrating may be performed on the washed product.

More specifically, regarding the method of isolating the stromal vascular fraction from adipose tissue, the literature [Aronowitz et al. SpringerPlus (2015) 4:713] and the like may be referred to.

This stromal vascular fraction is composed of various cells such as lymphocytes, adipocyte precursors, adipocyte-derived stromal cells, fibroblasts, endothelial cells, and pericytes (Stem Cell Res Ther 2017 Jun 15;8(1):145). When SVF is cultured, lymphocytes die and are removed, and various types of adherent stromal cells can be obtained (Cytotherapy 15, 641-648, 2013).

In the present disclosure, the stromal vascular fraction may be obtained from adipose tissue of an arbitrary mammal, more preferably may be obtained from adipose tissue of humans, mice, rats, rabbits, monkeys, pigs, horses, cows, sheep, antelopes, dogs, or cats, more preferably may be obtained from humans, mice, rats, or monkeys, and most preferably may be obtained from humans or mice.

In addition, in the present disclosure, the stromal vascular fraction may be obtained from arbitrary adipose tissue. The stromal vascular fraction may be obtained from subcutaneous fat tissue or visceral fat tissue, which is a fat tissue around organs, and may be obtained from brown adipose tissue or white adipose tissue.

In addition, in the present disclosure, the two-dimensional culture of the stromal vascular fraction isolated from adipose tissue refers to a two-dimensional monolayer culture of the stromal vascular fraction so that one side of the cell culture is attached to a culture surface such as a culture dish to grow.

In the present disclosure, the spheroids of the stromal vascular fraction isolated from adipose tissue are obtained in a spherical shape by three-dimensionally culturing the stromal vascular fraction isolated from adipose tissue, and various three-dimensional culture methods for obtaining such spheroids are known in the art. Examples of such methods include, the haning-drop cultures method (Biotechnol Bioeng 2003, 83(2):173-80; Trends Biotechnol 2004, 22(4):195-202; Crit Rev Oncol Hematol 2000, 36(2-3):107-22), a culture method using microwell plates with non-adhesive surface (Cancer Res 1977, 37(10):3639-43; In Vitro Cell Dev Biol Anim 2001, 37(10):656-67), a culture method using microfabricated microstructures (Biomed Microdevices 2008, 10(2):197-202; Tissue Eng 2007, 13(8):2087-94), a culture method using rotation bioreactor (In Vitro Cell Dev Biol Anim 1997, 33(6):459-66; Nat Med 1998, 4(8):901-7), a culture method using surface modified substrates or scaffolds (Tissue Eng 2007, 13(7):1455-68; Tissue Eng 2006, 12(5):1357-68), a culture method using external forces (Cell Transplant 2006, 15 (Cell Transplant 2006, 15(6):521-32; Biotechnol Bioeng 2007, 98(3):694-700), and a culture method using spheroid on a chip (Biomaterials 2007, 28(3):559-66; Biomaterials 2006, 27(36):6032-42) and the like. For more specific details regarding the three-dimensional culture method for obtaining spheroids by culturing the stromal vascular fraction isolated from adipose tissue, the literature [Korean J Otorhinolaryngol-Head Neck Surg 2020, 63(6):245-51] may be referred to.

In addition, in the present disclosure, loading the antigen on the dendritic cells causes the antigen peptide to be presented in the form of a complex with MHC on the surface of the dendritic cells by antigen processing. This is to increase the immune response of anti-viral, anti-microbial, anti-tumor, etc., by inducing the activity of T cells having a T cell receptor that selectively recognizes the presented antigen peptide. As a method for loading an antigen on the such dendritic cells, the peptide antigen itself is introduced and used, or a method for expressing antigenic proteins in dendritic cells by treating cell-penetrating peptides such as antigen-expressing genes such as RNA, antigen-expressing virus vectors, and Tat peptides of the human immunodeficiency virus (HIV) bound to antigens are known in the art, and as in the Examples of the present disclosure, Fe₃O₄-ZnO core-shell nanoparticles, FZ -NP can also be used. More specific methods for loading antigens into dendritic cells are described in Immunol Res. 2017, 65: 798-810] and the like.

In addition, in this disclosure, antigens may be virus-derived antigens, pathogenic microbial-derived antigens such as bacteria, and tumor-derived antigens may be arbitrary antigens derived from cancer cells, such as surface expression proteins, surface expression receptors, peptides or proteins inside tumor cells, or a lysate of tumor cells, and the like.

These tumor antigens are preferably expressed only in cancer cells or overexpressed in cancer cells compared to normal cells, and may be, for example, epidermal growth factor receptor variant III (EGFRvIII) expressed in glioblastoma, epidermal growth factor receptor (EGFR) overexpressed in anaplastic thyroid cancer, breast cancer, lung cancer, glioma, etc., metastin receptor overexpressed in papillary thyroid cancer, etc., ErbB-type receptor tyrosine kinases overexpressed in breast cancer, etc., human epidermal growth factor receptor 2 (HER2) overexpressed in breast cancer, bladder cancer, gallbladder cancers, cholangiocarcinomas, esophagogastric junction cancers, etc., tyrosine kinase-18-receptor (c-Kit) overexpressed in nutritive kidney carcinoma, etc., HGF receptor c-Met overexpressed in esophageal adenocarcinoma, CXCR4 or CCR7 overexpressed in breast cancer, etc., endothelin-A receptor overexpressed in prostate cancer, peroxisome proliferator activated receptor δ (PPAR-δ) overexpressed in rectal cancer, platelet-derived growth factor receptor α (PDGFR-α) overexpressed in ovarian cancer, etc., CD133 overexpressed in liver cancer, multiple myeloma, etc., carcinoembryonic antigen (CEA) overexpressed in lung cancer, colorectal cancer, stomach cancer, pancreatic cancer, breast cancer, rectal cancer, colon cancer, medullary thyroid cancer, etc., epithelial cell adhesion molecule (EpCAM) overexpressed in liver cancer, stomach cancer, colorectal cancer, pancreatic cancer, breast cancer, etc., disialoganglioside (GD2) overexpressed in neuroblastoma, etc., glypican 3 (GPC3) overexpressed in hepatocellular carcinoma, etc., prostate specific membrane antigen (PSMA) overexpressed in prostate cancer, etc., tumor-associated glycoprotein 72 (TAG72) overexpressed in ovarian cancer, breast cancer, colon cancer, lung cancer, pancreatic cancer, etc., disialoganglioside (GD3) overexpressed in melanoma, etc., human leukocyte antigen-DR (HLA-DR) overexpressed in blood cancer, solid cancer, etc., mucin 1 (MUC1) overexpressed in advanced solid cancer, etc., New York esophageal squamous cell carcinoma 1 (NY-ESO-1) overexpressed in advanced non-small-cell lung cancer, etc., latent membrane protein 1 (LMP1) overexpressed in nasopharyngeal neoplasms, etc., tumor-necrosis factor-related apoptosis-inducing ligand receptor (TRAILR2) overexpressed in lung cancer, non-Hodgkin's lymphoma, ovarian cancer, colon cancer, colorectal cancer, pancreatic cancer, etc., vascular endothelial growth factor receptor 2 (VEGFR2), an angiogenesis factor receptor, and hepatocyte growth factor receptor (HGFR) overexpressed in hepatocellular carcinoma, etc. The tumor antigens may also be CD44, CD166, etc., which are surface antigens of cancer stem cells. Many antigens overexpressed in cancer cells compared to normal cells are known in the art, and for more specific information regarding tumor antigens other than those exemplified above, reference may be made to the literature [Anne T Collins et al. Prospective Identification of Tumorigenic Prostate Cancer Stem Cells. Cancer Res. 2005 Dec 1;65(23):10946-51], the literature [Chenwei Li et al. Identification of Pancreatic Cancer Stem Cells. Cancer Res. 2007 Feb 1;67(3):1030-7], the literature [Shuo Ma et al. Current Progress in CAR-T Cell Therapy for Solid Tumors. Int J Biol Sci. 2019 Dec 7;15(12):2548-2560], the literature [Dhaval S Sanchala et al. Oncolytic Herpes Simplex Viral Therapy: A Stride Toward Selective Targeting of Cancer Cells. Front Pharmacol. 2017, 16; 8: 270] and the like.

The composition for enhancing immunity of the present disclosure can be used against arbitrary cancer cells expressing an antigen loaded on dendritic cells when the composition is used for anti-tumor purposes. Cancer cells include esophageal cancer, stomach cancer, colorectal cancer, rectal cancer, oral cancer, pharyngeal cancer, laryngeal cancer, lung cancer, colon cancer, breast cancer, cervical cancer, endometrial cancer, ovarian cancer, prostate cancer, testicular cancer, melanoma, bladder cancer, kidney cancer, liver cancer, pancreatic cancer, bone cancer, connective tissue cancer, skin cancer, brain cancer, thyroid cancer, leukemia, Hodgkin's disease, lymphoma, multiple myeloma, hematologic cancer, etc.

In the present disclosure, the term "anti-tumor" refers to the suppression or delay of pathological symptoms of cancer due to apoptosis of cancer cells, reduction of viability of cancer cells, suppression of proliferation of cancer cells, suppression of cancer metastasis, and suppression of cancer recurrence.

The composition for enhancing immunity of the present disclosure may be used in combination or in combination with an arbitrary approved anti-viral agent, arbitrary anti-microbial agent (i.e., antibiotic), or arbitrary anti-cancer agent. When used in combination or in combination with an anti-cancer agent, a such anti-cancer agent includes an arbitrary anti-cancer agent that is cytotoxic to cancer cells, such as a metabolic antagonist, an alkylating agent, a topoisomerase antagonist, a microtubule antagonist, a plant-derived alkaloid, arbitrary cytokine drug, arbitrary antibody medicines, arbitrary immune checkpoint inhibitor medicines, arbitrary cell therapy (car-T cell therapy, car-NK cell therapy) medicines, and the like. Specifically, taxol, nitrogen mustard, imatinib, oxaliplatin, gefitinib, bortezomib, sunitinib, carboplatin, cisplatin, rituximab, erlotinib, sorafenib, IL-2 drugs, INF-α drugs, INF-γ drugs, trastuzumab, blinatumomab, ipilimumab, pepbrolizumab, nivolizumab, atezolizumab, duvalumab, bevacizumab, cetuximab, Tisagenlecleucel (Cymria), axicaptagen ciloleucel (TAC, Yescata), etc., may be exemplified, and in addition to these exemplified anti-cancer agents, other anti-cancer agents known in the art may be used in combination or in combination with the composition of the present disclosure without limitation.

The composition of the present disclosure may be formulated by a conventional method known in the art according to an administration path, including a pharmaceutically acceptable carrier or excipient, and may be prepared as a pharmaceutical composition.

Such pharmaceutically acceptable carriers or excipients are not particularly toxic to the human body and do not impair the activity or characteristics of the drug and include lactose, dextrose, sucrose, sorbitol, mannitol, starch, acacia gum, calcium phosphorate, alginate, gelatin, calcium silicate, microcrystalline cellulose, polyvinylpyrrolidone, cellulose, water (e.g., saline and sterile water), syrup, methyl cellulose, methylhydroxybenzoate, propylhydroxybenzoate, talc, magnesium stearate, mineral oil, Ringer's solution, buffer solution, maltodextrin solution, glycerol, ethanol, dextran, albumin, or an arbitrary combination thereof. In particular, when the pharmaceutical composition of the present disclosure is formulated with a liquid solution as a suitable carrier or excipient, at least one component from components such as saline, sterile water, Ringer's solution, buffered saline, albumin injection solution, dextrose solution, maltodextrin solution, glycerol, ethanol, and the like may be used alone or in combination. Depending on the requirements, other conventional pharmaceutical additives such as anti-oxidants, buffers, and bacteriostats may be added and used.

The pharmaceutical composition of the present disclosure may be formulated as a parenteral administration agent, particularly an injection, and in this case, the parenteral administration agent may be prepared in the form of a unit dose ampoule or multiple doses. In particular, the pharmaceutical composition of the present disclosure may be formulated in the form of a solution, suspension, or the like.

The pharmaceutical composition of the present disclosure may be formulated in a unit dosage form suitable for administration in the body of a patient according to a conventional method in the pharmaceutical field and administered using an administration method commonly used in the art, in which the method by parenteral administration routes, such as skin, intralesional, intravenous, intramuscular, intraarterial, intramedullary, intrathecal, intraventricular, pulmonary, transdermal, subcutaneous, intraperitoneal, intranasal, gastrointestinal, topical, sublingual, vaginal, rectal route, kidney capsule, and the like may be used.

The dosage (effective amount) of the pharmaceutical composition of the present disclosure can be prescribed in various ways depending on factors such as formulation method, administration method, age, weight, sex, pathology, food, administration time, administration route, excretion rate, and reaction sensitivity of the patient. Those skilled in the art can appropriately determine the dosage in consideration of these factors. In a preferred embodiment, the pharmaceutical composition of the present disclosure is prepared as an injection in the form of a unit dose, and when prepared as an injection in the form of a unit dose, the stromal vascular fraction spheroids or dendritic cells included per unit dose of the pharmaceutical composition of the present disclosure may be in a range of 10² to 10⁸ cells.

In another aspect, the present disclosure relates to a method for enhancing immunity including administering to a subject, such as a patient, an effective amount of the pharmaceutical composition for enhancing immunity of the present disclosure as described above. When the composition for enhancing immunity of the present disclosure is used for anti-viral purposes by including dendritic cells loaded with viral antigens, the method for enhancing immunity may be used for treating or preventing viral infections. When the composition for enhancing immunity of the present disclosure is used for anti-microbial purposes by including dendritic cells loaded with antigens of pathogenic microorganisms such as bacteria, the method for enhancing immunity may be used for treating or preventing infection of pathogenic microorganisms. When the composition for enhancing immunity of the present disclosure is used for anti-tumor purposes by including dendritic cells loaded with tumor antigen, the method for enhancing immunity may be anti-tumor (cancer treatment or prevention) use.

The method for enhancing immunity of the present disclosure becomes possible by allowing spheroids of the stromal vascular fraction and antigen-loaded dendritic cells contained in the composition for enhancing immunity of the present disclosure to activate T cells specific for the antigen, thereby providing anti-viral, anti-microbial, and anti-tumor activity. Therefore, the method for enhancing immunity of the present disclosure can be applied to the arbitrary viral infection having such an antigen, a microbial infection having such an antigen, and arbitrary carcinoma having such an antigen.

When the method for enhancing immunity of the present disclosure is used for cancer treatment or prevention for anti-tumor use, the method for enhancing immunity may be used in combination with other cancer treatment methods without limitation. For example, cytotoxic anti-cancer drugs, cytokine drugs, antibody drugs, immune checkpoint inhibitor drugs, car-T cell therapy (car-NK cell therapy) drugs, radiation therapy, and surgical therapy may be used in combination before and after administration or in a simultaneous administration mode of the composition of the present disclosure.

The effective amount in the treatment method of the present disclosure refers to the amount of the composition of the present disclosure, which may exhibit intended medical effects such as cancer treatment or prevention effects, when the composition of the present disclosure is administered during the administration period according to the suggestion of a medical professional, etc. As described above, this effective amount can be appropriately determined by those skilled in the art, such as medical experts, according to the patient's age, weight, gender, and pathological condition.

In another aspect, the present disclosure can be identified as a method for enhancing immunity that includes administering a composition for enhancing immunity containing a stromal vascular fraction isolated from adipose tissue, particularly spheroids and dendritic cells of the stromal vascular fraction as active ingredients, or administering the composition to a subject such as a patient in an effective amount.

As described above, in the Examples below, the present inventors confirmed that the interaction between dendritic cells and T cells was enhanced when the spheroids of the stromal vascular fraction isolated from adipose tissue were transplanted together with dendritic cells in vivo. In addition, when the two-dimensional culture of the stromal vascular fraction isolated from adipose tissue was co-cultured with dendritic cells in vitro, it was confirmed that T cells were effectively activated by promoting the activation of dendritic cells and improving the survival rate of dendritic cells.

These results show that when spheroids and dendritic cells of the stromal vascular fraction isolated from adipose tissue are administered together, the immune response, specifically the cell-mediated immune response, and more specifically, the immune response by T cells is enhanced.

As described above with respect to the composition for enhancing immunity of the present disclosure including a vascular matrix fraction and dendritic cells loaded with an antigen or a method for enhancing immunity using such a composition, the composition for enhancing an immune response or a method for enhancing immunity by administering such a composition of the present disclosure can also be used for anti-viral, anti-microbial, or anti-tumor purposes, and these compositions and methods can also be embodied and used as described above.

In another aspect, the present disclosure relates to a dendritic cell maturation composition including a stromal vascular fraction isolated from adipose tissue, particularly a spheroid of the stromal vascular fraction, as an active ingredient.

As described above, in the following Examples, the present inventors confirmed that spheroids of stromal vascular fraction isolated from adipose tissue highly express various factors (Spp1, Ccl8, Cxcl5, Postn, Cxcl16, Ccl17, etc.) such as chemokines that attract and activate immune cells, and also highly express osteopontin, a factor known to regulate the maturation and survival of dendritic cells, the two-dimensional culture of the stromal vascular fraction promotes the activation of dendritic cells and improves the survival rate of dendritic cells when co-cultured spheroids of stromal vascular fraction with dendritic cells in vitro.

These results can be said to show that the spheroids of the stromal vascular fraction isolated from adipose tissue can induce the maturation of dendritic cells and further increase the activation and survival rate.

In order to utilize dendritic cells for anti-tumor and immunotherapy, etc., it is essential to develop a technology to differentiate and manufacture dendritic cells ex vivo, as well as a maturation technology to effectively induce a T-cell immune response. As described above, mature dendritic cells express a large number of cytokines, such as IL-12, IL-10, and TNF, and these cytokines are directly involved in the induction of T cell immune responses. These changes through maturation greatly increase the ability of dendritic cells to induce T cell immune responses.

The dendritic cell maturation composition of the present disclosure may further include IFN-α, IL-1β, IL-6, IL-3, TNF-α, IFN-γ, PGE2, OK432 (Picibanil) and the like, known in the art as a maturation factor for dendritic cells, and may be added to Roswell Park Memorial Institute (RPMI) 1640 medium, X-VIVO15 which is a serum-free medium, and the like and used.

The dendritic cell maturation composition of the present disclosure may be prepared as a pharmaceutical composition and administered to a subject such as a patient, or used for the purpose of maturation and proliferation of dendritic cells ex vivo.

### Advantageous Effects

As described above, the present disclosure may provide a composition for enhancing an immune response using the activation function of dendritic cells of the stromal vascular fraction isolated from adipose tissue, specifically, a composition for enhancing an immune response including the stromal vascular fraction isolated from adipose tissue, particularly spheroids of stromal vascular fraction and dendritic cells loaded with antigen as active ingredients, and a composition for enhancing an immune response including the stromal vascular fraction isolated from adipose tissue, particularly spheroids of stromal vascular fraction and dendritic cells as active ingredients.

The composition of the present disclosure can be used for anti-viral use, anti-microbial use, anti-tumor use, and the like.

### Description of Drawings

FIGS. 1 to 5 are results showing that when a stromal vascular fraction isolated from adipose tissue is cultured, the stromal vascular fraction has characteristics and functions similar to those of lymph node stromal cells;
FIG. 6 is a result showing that the spheroids of the stromal vascular fraction isolated from adipose tissue may form blood vessels that become the migration channels of immune cells;
FIGS. 7 to 11 show that the spheroids of the stromal vascular fraction isolated from adipose tissue attract dendritic cells and promote the interaction of dendritic cells with T cells, and the T cells are mostly differentiated into CD4+ effector T cells to induce an immune response;
FIGS. 12 and 13 show that a two-dimensional culture of a stromal vascular fraction isolated from adipose tissue or a spheroid thereof improves the survival rate of dendritic cells and enhances the immune response of antigen-specific T cells together with dendritic cells loaded with antigen;
FIG. 14 are results showing that a spheroid of a stromal vascular fraction isolated from adipose tissue reduces the size of the tumor and improves the survival rate of mice when spheroids are administered to mice transplanted with B16 melanoma cells (B16-OVA) expressing tumor antigen (OVA) together with dendritic cells loaded with tumor antigen (OVA); and
FIG. 15 is a conceptual diagram of the present disclosure.

### Best Mode

Hereinafter, the present disclosure will be described with reference to embodiments. However, the scope of the present disclosure is not limited to the examples.

### Example

### 1. Samples and test methods

### 1.1 Mouse

C57BL/6 wild-type mice (Koatech, Seoul, Republic of Korea) and C57BL/10NAGCSAni-(KO) Rag2(H-2b) mice (Taconic Biosciences, NY, US) were raised in a specific pathogen-free facility located at Seoul National University Medical School. All mice were male and mice aged 6 to 12 weeks were used. All mouse experiments were approved by the Institutional Animal Care and Use Committee (IACUC) of Seoul National University (IACUC Number: SNU-150115-2-7, SNU-160212-2-7, SNU-171123-4-7).

### 1.2 Isolation of stromal vascular fraction (SVF) and spheroid culture

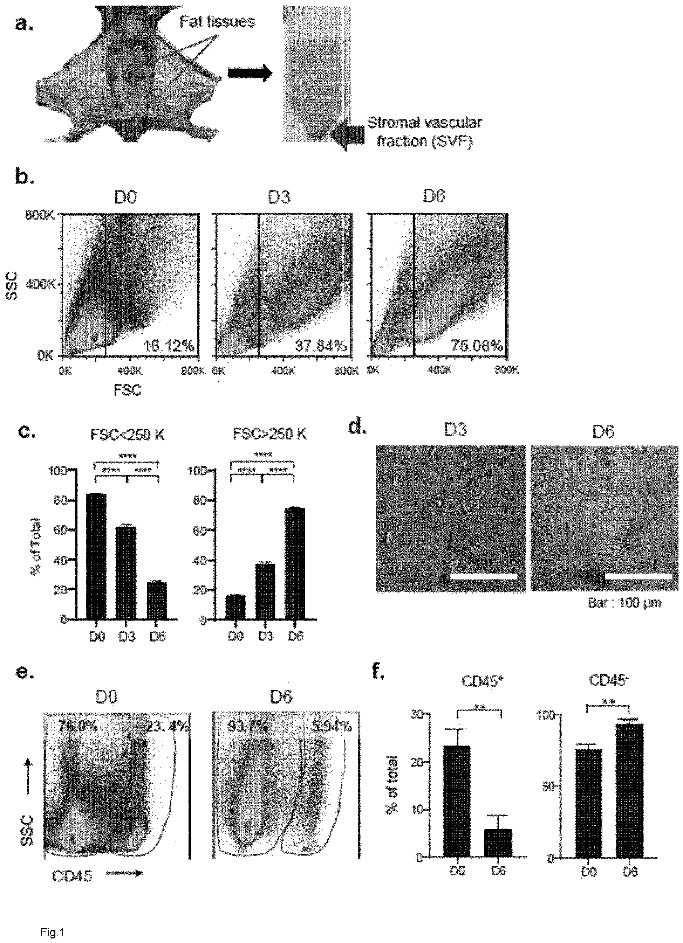

After collecting and chopping adipose tissue from the intestines (Viscera) and skin (Skin), the tissue was cultured in a sterilized Hank's balanced salt solution (Intron, Seoul, Republic of Korea) that includes 3% bovine serum albumin (BSA) (MP biomedicals, CA, US) containing 0.8 mg/ml collagenase and 100ug/ml DNase I, 1.0 mM CaCl₂ (Sigma-Aldrich, MO, US), and 0.8 mM MgCl₂ (Sigma-Aldrich, MO, US) for 1 hour at 170rpm in a shaking incubator at 37°C. After centrifugation at 500 g for 3 minutes, the supernatant was removed and the stromal vascular fraction (SVF), which was a pellet, was isolated (FIG. 1A) and then cultured in Dulbecco's Modified Eagle Medium (Welgene, Daegu, Republic of Korea) containing 10% fetal bovine serum (Gibco, MA, US) and 1% penicillin/streptomycin (Gibco, MA, US). To make spheroids, SVF cultured for 6 to 10 days was treated with trypsin and counted. The cultured SVF was dispensed on a spheroid film (Incyto, Cheonan, Republic of Korea). It was confirmed that 361 SVF-spheroids (19X19) were formed on the spheroid film, and after culturing for more than two days, SVF-spheroids were used in the experiment.

### 1.3 Production of recombinant ZnO-binding peptide (ZBP)-ovalbumin (OVA) protein

To produce the recombinant ZnO-binding peptide (ZBP)-ovalbumin (OVA) protein (ZBPOVA), the genomic DNA of the B16MO5 cell line was first extracted according to the manufacturer's instructions using DNeasy Blood & Tissue Kits (Qiagen, Hilden, Germany). The extracted DNA is amplified by PCR (Forward primer: 5'-TTT GAA TTC ATG GGC TCC ATC GGT GCA-3' SEQ ID NO: 1, Reverse primer: 5'-AAA CTC GAG AGG GGA AAC ACA TCT GCC-3', EcoRI, XhoI restriction sites underlined) and cloned from the pET23d-ZBP vector encoded with 3xZBP (BamHI- PHRKGGDARPHRKGGDARPHRKGGDA-EcoRI, SEQ ID NO: 3). The cloned vector pET23d-ZBPOVA was transformed into the E. coli HIT Competent BL21 strain (RBCBioscience, New Taipei City, Taiwan) according to the manufacturer's instructions. ZBPOVA was purified by a protocol appropriately adapted from the previously described methods (Nature methods 3, 55-64, 2006; Biochemistry and Molecular Biology Education 39, 28-37, 2011). Briefly, an E. coli culture in which protein expression was induced by isopropylthiogalactoside (IPTG; 0.1 mM) was treated with ice-cold buffer (300 mM NaCl, 50 mM phosphate, pH = 8.0), in which lysozyme (1 mg/ml) and protease inhibitor cocktail is added, for 30 min and further dissolved by sonication. The lysate was centrifuged, the supernatant was collected, filtered, and purified by column using AKTAstart (GEHealthcare, IL, US) and HisTrap^{™} FF (GEHealthcare, IL, US). Proteins were dialyzed in PBS at 4°C overnight. Endotoxin was removed from the purified protein using Triton X-114 as previously described (Microbial cell factories 10, 57, 2011). Endotoxin contamination of purified proteins was determined using the Pierce LAL Chromogenic Endotoxin Quantification Kit (Thermo Fisher Scientific, MA, US) according to the manufacturer's instructions. Purified ZBPOVA protein was quantified by SDS-PAGE and stored at -80°C until further use.

### 1.4 Bone marrow-derived dendritic cells isolation, antigen delivery and maturation

As previously described (Nat Nanotechnol 6, 675-682, 2011), bone marrow was isolated and differentiated into dendritic cells. Briefly, the bone marrow of Rag2-knock-out mice was isolated and cultured in Iscove's modified Eagle medium (IMDM) (Gibco, MA, US) supplemented with 10% FBS, 1.5 ng/ml recombinant mouse GMCSF (PeproTech, NJ, US), 1.5 ng/ml mouse IL-4 (PeproTech, NJ, US), 1% penicillin/streptomycin, 50 µg/ml gentamicin (Gibco, MA, US), 2 mM L-glutamine (Gibco, MA, US), 50 nM β-mercaptoethanol (Gibco, MA, US). Immature dendritic cells were cultured for 6 to 8 days. The medium was changed every other day. Fe₃O₄-ZnO core-shell nanoparticles (FZ-NP) were used to transport internal antigens to immature bone marrow-derived dendritic cells (BMDCs). 100ug FZ-NPs were washed 3 times with PBS, and FZ-NPs were cultured at room temperature for 1 hour to bind with 20ug ZBP-OVA. Then, ZBP-OVA and FZ-NPs (NP-OVA) were washed 3 times with IMDM. Next, 1×10⁶ dendritic cells were cultured with NPOVA at 37°C for 1 hour. For the maturation process, dendritic cells were treated with 1 ug/ml lipopolysaccharide (LPS) for 16 to 18 hours. Dendritic cells matured by LPS were washed 3 times with IMDM before the experiment.

### 1.5 Flow cytometry and antibodies

Cells were blocked with a super-block solution being configured with 10% goat serum (Thermo Fisher Scientific, MA, US), 10% rat serum (Thermo Fisher Scientific, MA, US), 10% mouse serum (Sigma-Aldrich, MO, US), 10 ug/ml anti-CD16/CD32 (2.4G2) antibody (BD Pharmingen, NJ, US). The surface marker was then stained with APC/Cy7-conjugated anti-CD45, BV405-conjugated anti-CD11c, alexa488-conjugated anti-CD11c, APC-conjugated anti-B220, PE-conjugated anti-CD11b, APC-conjugated anti-PDPN, FITC-conjugated antiICAM-1, PE/Cy7-conjugated anti-CD31, PE-conjugated anti-LTβR, FITC-conjugated anti-VCAM-1, PE-conjugated antiCD140α, PerCP/Cy5.5-conjugated anti-CD44, PE/Cy7-conjugated anti-CD86, APC-conjugated antiI-Ab, BV605-conjugated anti-Ki67, PE-conjugated anti-DC-SIGN (Biolegend, CA, US), PE-conjugated anti-CD3, BV421-conjugated anti-CD3, PerCP-conjugated anti-CD4, FITC-conjugated anti-CD8, APC-conjugated antiGr-1, BV605-conjugated anti-CD62L, FITC-conjugated anti-I-Ab, PE-conjugated anti-CD40 (BD Pharmingen, NJ, US), PE/Cy7-conjugated anti-CD4, eFlour 450-conjugated anti-CD3, PE/Cy7-conjugated anti-F4/80, APC-conjugated anti-CD80 (eBioscience, CA, US) in ice for 30 minutes. Live cells were separately stained with Zombi Aqua (Biolegend, CA, US) or 7-aminoactinomycin D (7-AAD) (BD Pharmingen, NJ, US). Cells were measured using a LSRFortessa X-20 flow cytometer, a BD LSRII flow cytometer (BD Pharmingen, NJ, US), and a CytoFLEXS (Beckman Coulter, CA, US). Data were analyzed with FlowJo software (Tree Star, Ashland, OR, USA).

### 1.6 Transplanted with kidney capsules

Transplantation of SVF-spheroids or dendritic cells into the kidney capsule was performed as previously mentioned (Biochem Biophys Res Commun 514, 1081-1086, 2019). Briefly, the mouse was anesthetized, the hair on the side to be operated on was removed, the skin was disinfected, the incision was made, and the kidneys were exposed to the outside. The kidney capsule surface was torn with a 30-gauge needle (BD Pharmingen, NJ, US), and the graft was slowly injected using a threaded plunger (Hamilton, NV, US). The incision was sutured with a high-temperature cautery (Bovie Medical Corporation, NY, US). The kidney was put back into the body, and the skin was sutured.

### 1.7 Quantitative PCR

RNA extraction was performed using Trizol reagent according to a standard protocol. Complementary DNA (cDNA) was sequentially synthesized using a reverse transcript premix kit (Intron, Seoul, Republic of Korea). To determine whether SVF can secrete major chemokines of lymph node stromal cells, mRNA expression was measured by quantitative PCR (qPCR) using SYBR Green master mix (Life Technologies, CA, US) and CFX Real Time PCR Detection System (Bio-Rad Laboratories, CA, US). Each sample was conducted in two repetitive experiments. Relative mRNA expression was calculated based on the value of β-actin (Actb). Primers are summarized in Table 1.

**Table 1.**

| Gene | Forward Primer: | Reverse Primer: |
|---|---|---|
| CCL19 | CCTGGGAACATCGTGAAAGC (SEQ ID NO: 3) | TAGTGTGGTGAACACAACAGC (SEQ ID NO: 4) |
| CCL21 | GTGATGGAGGGGGTCAGGA (SEQ ID NO: 5) | GGGATGGGACAGCCTAAACT (SEQ ID NO: 6) |
| CXC13 | GGCCACGGTATTCTGGAAGC (SEQ ID NO: 7) | GGGCGTAACTTGAATCCGATCTA (SEQ ID NO: 8) |
| CXCL12 | CATCAGTGACGGTAAACCAG (SEQ ID NO: 9) | CACAGTTTGGAGTGTTGAGG (SEQ ID NO: 10) |
| Actb | TGTTACCAACTGGGACGACATG (SEQ ID NO: 11) | GGGGTGTTGAAGGTCTCAAAC (SEQ ID NO: 12) |

### 1.8 mRNA sequencing analysis

To identify the gene expression characteristics of SVF and SVF-spheroids, CD45-cells were classified from SVF, and total RNA was isolated using anti-CD45 microbeads and magnetic cell separation (MACS). The quality of RNA was measured with the Agilent 2100 bioanalyzer using the RNA 6000 Nano Chip (Agilent Technologies, Amstelveen, Netherlands), and the amount of RNA was quantified using the ND-2000 Spectrophotometer (Thermo Fisher Scientific, MA, US). Libraries were generated from total RNA using the SMARTer Stranded RNA-Seq Kit (Clontech Laboratories, CA, US). Polyadenylated mRNA was specifically isolated using Poly(A) RNA Selection Kit (LEXOGEN, Vienna, Austria), and shearing for cDNA synthesis, fragmentation, and PCR amplification were sequentially performed. Average fragment size was measured with an Agilent 2100 bioanalyzer, and quantification was assessed with a StepOne Real-Time PCR System (Life Technologies, CA, US). Libraries were sequenced with a paired-end 100-bp reading by HiSeq 2500 (Illumina, CA, US). The sequencing data were tabulated using TopHat. Data were analyzed with ExDEGA (E-biogen, Seoul, Republic of Korea) and visualized with Prism 8 (GraphPad, CA, US).

### 1.9 Enzyme-linked immunosorbent assay (ELISA)

The expression of CCL21 and osteopontin at the translational level in the cell culture supernatant was measured with an ELISAM kit purchased from Research and Diagnostic Systems (MN, US) and Lifespan Biosciences (WA, US), respectively, according to the manufacturer's instructions.

### 1.10 chemotaxis assay

In order to confirm the chemoattractive ability of SVF-spheroids, a chemotaxis assay was performed using a µ-Slide Chemotaxis kit (Cat#80326; Ibidi, Germany) according to the manufacturer's instructions. 1.5 mg/ml Type I Collagen solution (Corning, NY, US) was injected into the observation area. SVF-spheroids in the medium or medium were injected into the left space, and splenocytes were inserted into the right space. Live cell imaging was taken with an FV1000 (Olympus Corporation, Tokyo, Japan) during a 24-hour culturing period.

### 1.11 Co-culture of stromal vascular fraction (SVF) and dendritic cells (DC) or splenocytes

In a 24-well plate, 1×10⁶/well dendritic cells, 1x10⁶/well splenocytes, 1×10⁶/well mature dendritic cells (mature DC), or 2x10⁴/well SVF cultured with 1×10⁶/well splenocytes were prepared, and co-cultured. After culturing, cells were analyzed using flow cytometry.

### 1.12 In vitro and in vivo imaging

SVF cultured in vitro was observed with a microscope (Olympus corporation, Tokyo, Japan) and VisiView software (Visitron systems, Puchheim, Germany). To confirm the structure resulting from the kidney capsule transplantation in vivo, the grafts were collected two weeks after surgery, placed in frozen section media (Leica Biosystems, IL, US), and quickly frozen in liquid nitrogen. Samples were made into frozen sections with a thickness of 5 to 6 um and stored at -80°C until the experiment. For histological analysis, hematoxylin-eosin (H&E) staining was performed. For confocal imaging, section samples were fixed in 4% paraformaldehyde and stained with FITC-labeled anti-CD3, FITC-labeled anti-CD8 (BD Pharmingen, NJ, US), Alexa 647-labeled anti-CD31, Alexa 594- labeled anti-PDPN, Alexa 647-labeled anti-CD11c, Alexa 647-labeled anti-CD4 (Biolegend, CA, US), Alexa 594-labeled anti-VEGFR3 (Bioss Antibodies, MA, US), 4',6-diamidino-2-phenylindole (DAPI; Thermo Fisher Scientific, MA, US). Confocal images were taken with FV3000 (Olympus Corporation, Tokyo, Japan) and analyzed with IMARIS version 9.3 (Bitplane, Z rich, Switzerland) .

### 1.13 Evaluation of antigen-specific immune responses

To evaluate whether injection of SVF-spheroids, dendritic cells, each or a mixture thereof effectively induces an antigen-specific immune response, each combination (1x10⁶ dendritic cells/mouse or 361 SVF-spheroids/mouse or both) were transplanted into the kidney capsule of each mouse once per different weeks, for a total of two times. The dendritic cells injected in each group were intracellularly ingested with NP-OVA and then transplanted, and the same number of NP-OVA was injected into the SVF-spheroid-only transplanted group. Five weeks after the first immunization, splenocytes isolated from each mouse were cultured with OVA 257-264 peptides and OVA 339-323 peptides (InvivoGen, CA, US) presented by H-2K^{b} major histocompatibility complex (MHC) I and I-A^{b} MHC II, respectively, to diffuse OVA-specific T cells. After culturing for 24 hours, the cells were stained with anti-MHC tetramer at 4°C for 30 minutes, and cell surface staining was performed for an additional 30 minutes, followed by performing flow cytometry assay. APC-labeled tetramer SIINFEKL-H-2K^{b} and PE-labeled tetramer AAHAEINEA-IA^{b} were provided by the National Institutes of Health Tetramer Core Facility in the United States.

### 1.14 Tumor inoculation and anti-tumor effect test

For tumor inoculation, OVA-expressing malignant melanoma B16MO5 (5x10⁴cells/mouse) were injected into the left side of the mouse. After 7 days, each combination (1×10⁶ dendritic cells/mouse, 361 SVF-spheroids/mouse) was inserted under the kidney capsule three times a week. Each group consisted of 5 mice. Tumor size and survival rates were evaluated to investigate the anti-tumor effect. Tumor size was measured every 2 to 3 days. [tumor volume (mm)³ = 1/2 × [(short diameter) × (long diameter)²] was calculated. If the tumor size grew more than 2000 mm3, the mouse was considered dead.

### 1.15 Statistical analysis

The data were analyzed with GraphPad Prism software for statistical analysis. RNA sequencing was performed with ExDEGA version 2.5.

### 2. Results of Experiments

### 2.1 Characteristics of SVF isolated from adipose tissue

SVFs extracted from adipose tissue on the first 0 days (D0) were mostly small cells (FSC<250K, FSC: forward scatter, SCC: side scatter) in a circular shape, but when cultured (D3: 3rd day of culture, D6: 6th day of culture), SVFs extracted from adipose tissue were replaced by large (FSC>250K) long-shaped cells (FIGS. 1B to 1D). When flow cytometry was performed to confirm these cell changes in detail, it was confirmed that the CD45+ hematopoietic cell population decreased when cultured (23.4% → 5.94%) and the non-hematopoietic cell population increased (76.0% → 93.7%) (FIGS. 1E and 1F).

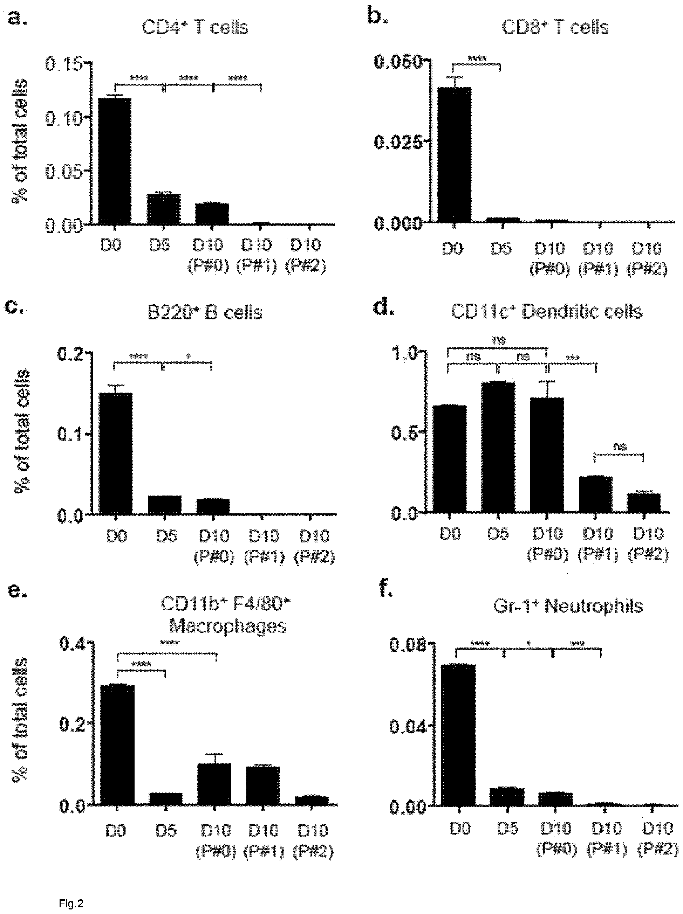

As a result of analyzing the cell types according to these cell changes through marker analysis, CD4+ T cells, CD8+ T cells, and B220+ B cells decreased rapidly, and CD11c+ dendritic cells were maintained up to 10 days of culture (FIGS. 2A to 2D), and CD11b+ F4/80+ macrophages and Gr-1+ neutrophil cells decreased (FIGS. 2E and 2F). In FIG. 2, P#0, P#1, and P#2 denote passage numbers 0, 1, and 2, respectively.

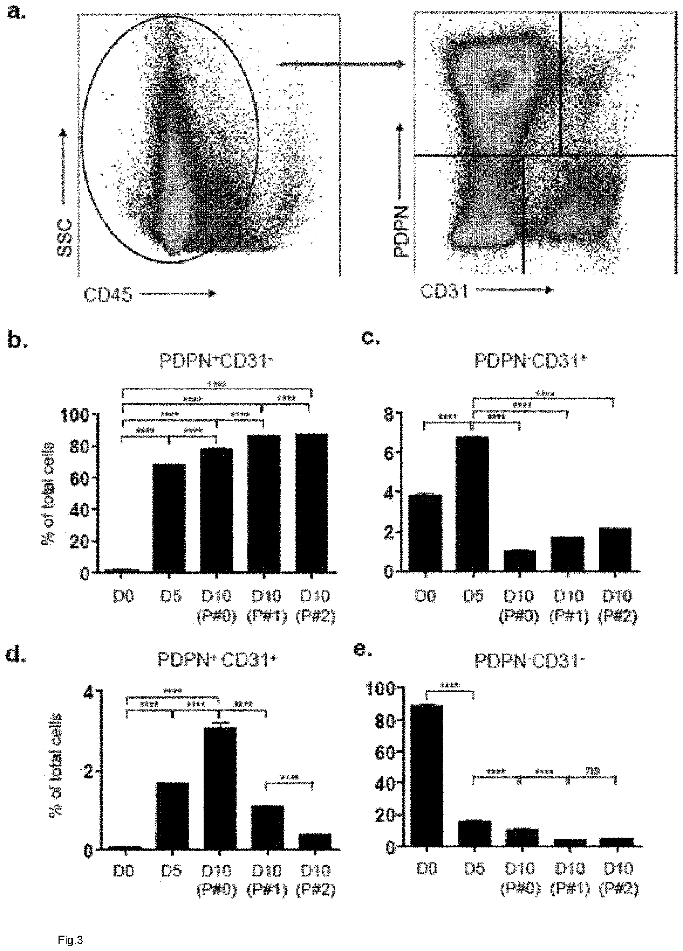

A subtype of lymph node stromal cells uses PDPN and CD31 markers to classify PDPN+ CD31- as fibroblastic reticular cells (FRC), and PDPN+ CD31+ as lymphatic endothelial cells (LECs), and PDPN- CD31+ as blood endothelial cells (BECs). When SVF isolated from adipose tissue is cultured and flow cytometric analysis is performed, the CD45- cell group is the phenotype of lymph node stromal cells, it was confirmed that the CD45- cell group showed characteristics that were divided into PDPN+ CD31-, PDPN+ CD31+, and PDPN- CD31+ similar to the phenotype of lymph node stromal cells (FIGS. 3A to 3E). In particular, it was confirmed that SVF showed a phenotype of FRC (PDPN+ CD31-) of 60% or more when cultured for 5 days or more, and 80% or more when cultured for 10 days or more (FIG. 3B).

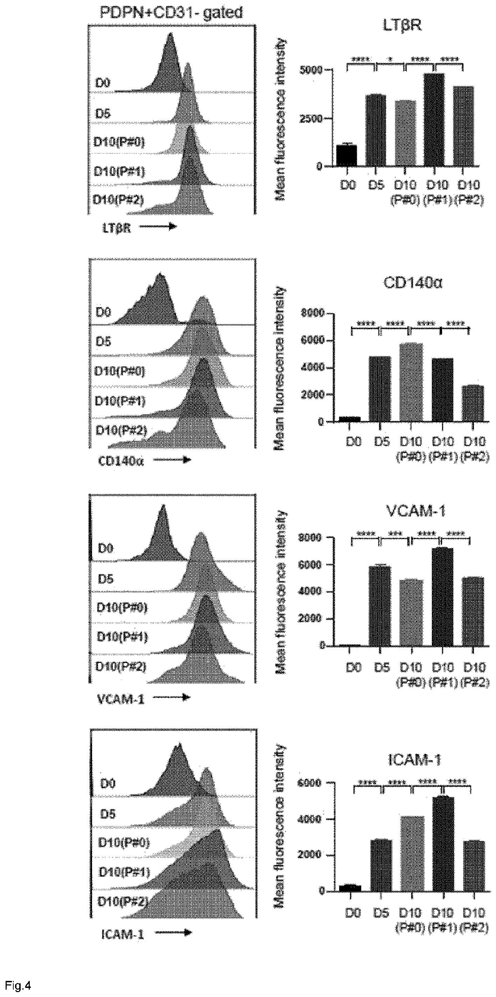

As a result of additional identification of SVFs with a PDPN+ CD31- FRC-like phenotype using FRC-specific markers, it was confirmed that these cells express FRC-specific markers such as lymphotoxin β receptor (LTβR), platelet-derived growth factor α (CD140α), vascular cell adhesion molecule-1 (VCAM-1), and intracellular adhesion molecule-1 (ICAM-1) (FIG. 4). In FIG. 4, P#0, P#1, and P#2 denote passage numbers 0, 1, and 2, respectively.

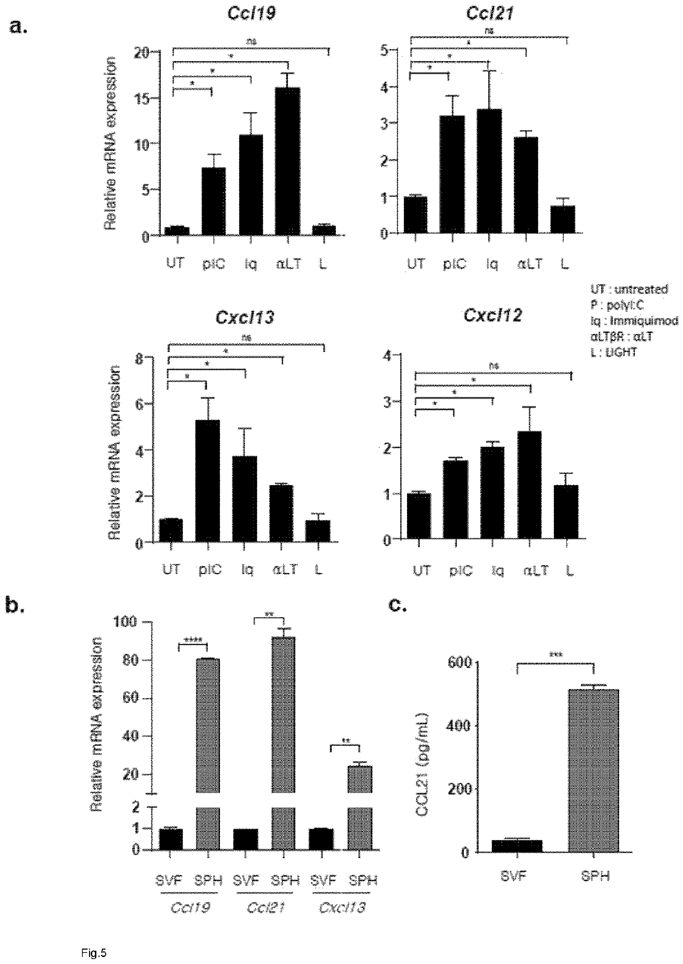

In addition to these phenotypes, in order to investigate whether SVF is functionally similar to lymph node stromal cells, various stimulators, such as TLR3-agonist (PolyI:C, plC), TLR7-agonist (Imiquimod), αLTβR (αLT) and LTβR ligands (anti-LTβR, LIGHT, L ) were treated for 24 hours, and when mRNA was quantified by quantitative PCR, it was confirmed that Ccl19, Ccl21, Cxcl13, and Cxcl12, which are representative chemokines secreted by lymph node stromal cells, were expressed (FIG. 5A). It was confirmed that when cultured in the form of spheroids, which is a three-dimensional cell culture method (SVF spheroids, SPH), much more chemokines, such as Ccl19, Ccl21, and Cxcl13, may be expressed compared to the two-dimensional cultured SVF (FIG. 5B).

The above results can be said to show that SVF isolated from adipose tissue is composed of cells similar to lymph node stromal cells in phenotype and function.

### 2.2 Angiogenic capacity of SVF spheroids

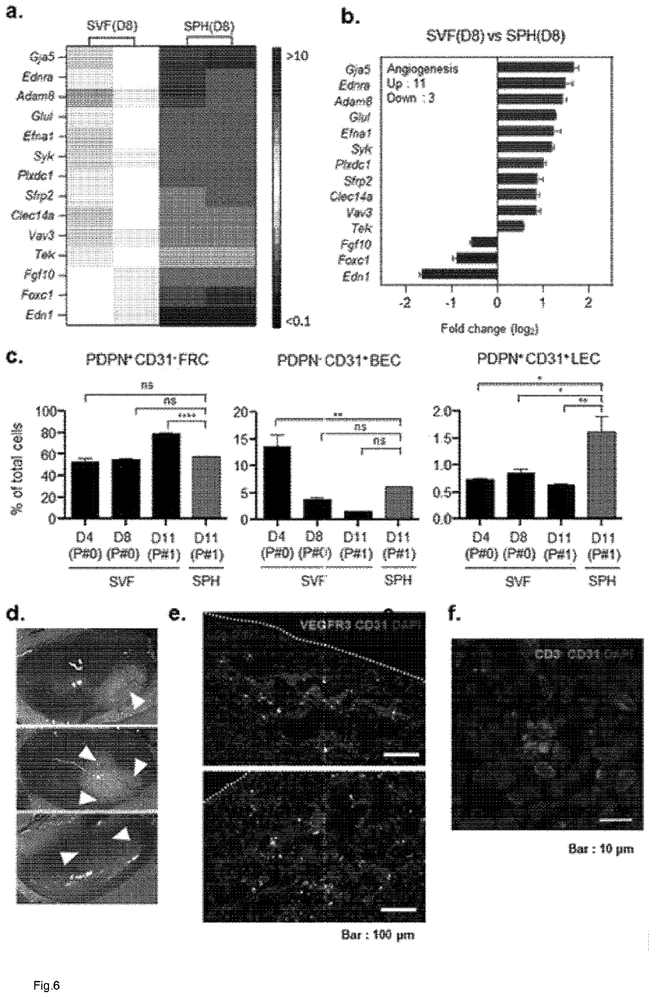

When comparing the gene expression of SVF spheroids (SPH) cultured for 8 days in a three-dimensional culture method (D8) and SVF (SVF) cultured in a two-dimensional culture method (monolayer) by mRNA analysis, SVF When cultured with spheroids, it was confirmed that the expression of 11 genes related to angiogenesis increased when cultured with SVF spheroids (FIGS. 6A and 6B), and CD31 cells (PDPN- CD31+ BEC, PDPN+ CD31+ LEC) increased when flow cytometry was performed (FIG. 6C). In FIG. 6, >10 means that the fold change value of gene expression is more than 10 times the average of the control group (SVF(D8)), <0.1 means less than 0.1 times, and P#0 and P#1 denote passage numbers 0 and 1, respectively.

In order to actually check the blood vessel formation ability in vivo, when SVF spheroids were transplanted into the kidney capsule of a mouse, it was confirmed that blood vessels were formed on the graft surface 2 weeks after transplantation (FIG. 6D), and the generated blood vessels were positive for VEGFR3 and CD31, which are lymphocytes and endothelial cell markers, respectively (FIG. 6E). In addition, CD3+ T cells appeared to migrate through interaction with CD31+ cells through frozen section images (FIG. 6F).

The above results can be said to show that SVF spheroids can form blood vessels that serve as migration channels for immune cells.

### 2.3 The ability of SVF spheroids to attract dendritic cells and enhance the interaction between T cells and dendritic cells

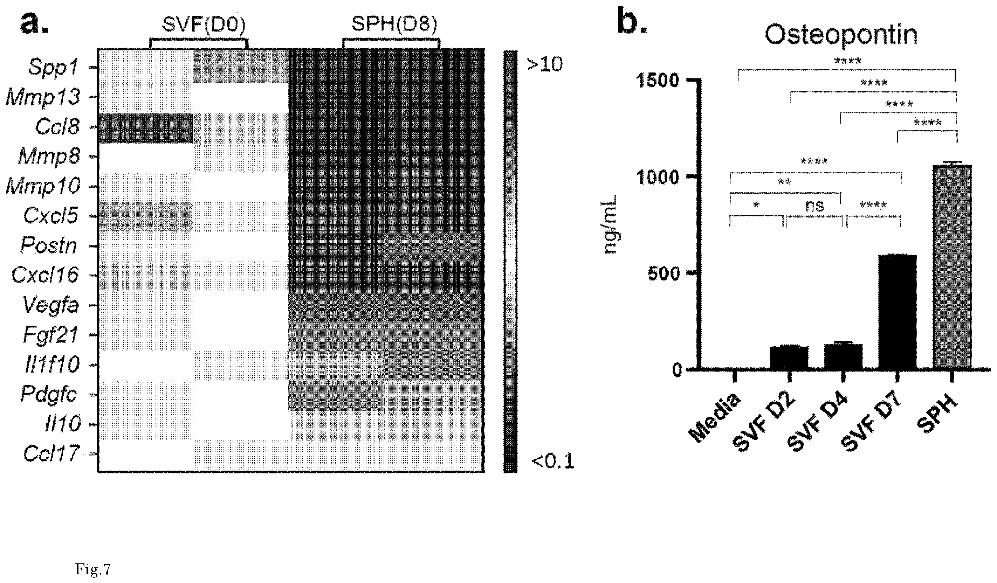

As a result of confirming the gene expression changes of chemokines, cytokines, and growth factors in three-dimensional cultured SVF spheroids (SPH) through mRNA analysis, it was confirmed that on day 0 (D0), the expression of factors that attract or activate immune cells increased statistically significantly (p value <0.05) compared to the two-dimensional culture SVF (FIG. 7A). In FIG. 7A, >10 means that the fold change value of gene expression is 10 times more than the average of the control (SVF(D0), and <0.1 means that the fold change value of gene expression is 0.1 times less than the average of the control (SVF(D0). Specifically, the expression of vascular endothelial growth factor α (Vegfa) gene and platelet-derived growth factor C (Pdgfc) gene, which are factors inducing angiogenesis, increased, the expression of Mmp 8, 10 and 13 (Matrix metalloproteinase 8, 10, 13) genes, which open the cell migration channel by decomposing the extracellular matrix, also increased, and the expression of Spp1, Ccl8, Cxcl5, Postn, Cxcl16, Ccl17 genes, which are factors that attract and activate immune cells, also increased. In particular, the expression of the gene Spp1 for osteopontin, a factor known to control the maturation and survival of dendritic cells, was increased, and quantification of osteopontin in the culture supernatant confirmed that the concentration of osteopontin in SVF spheroids increased very high (FIG. 7B).

These results can be said to show that SVF spheroids express various factors that attract and activate immune cells.

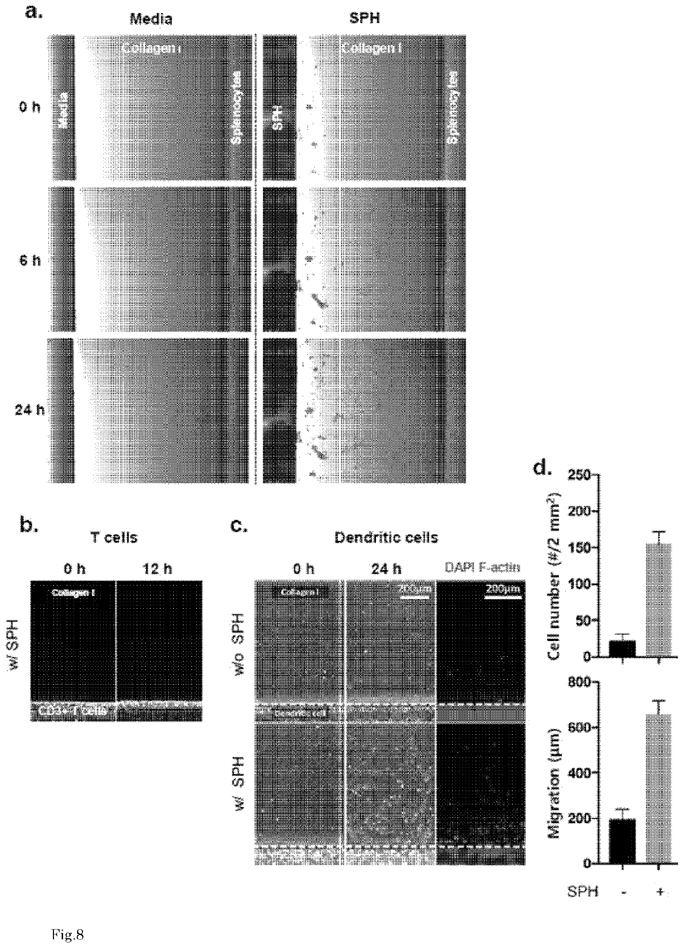

In order to confirm that the chemokines secreted from the SVF spheroids can actually attract immune cells, a migration assay was performed using the collagen layer for 24 hours, and as a result, it was confirmed that the splenocytes on the right side migrated toward the SVF spheroid (SPH) on the left side over time (FIG. 8A). When migration assay was performed on CD3+ T cells and bone marrow-derived dendritic cells (BMDCs) for the immune cell attraction ability of SVF spheroids, it was confirmed that CD3+ T cells did not migrate, but BMDCs, which are dendritic cells, migrated toward the SVF spheroids (FIGS. 8B, 8C, and 8D).

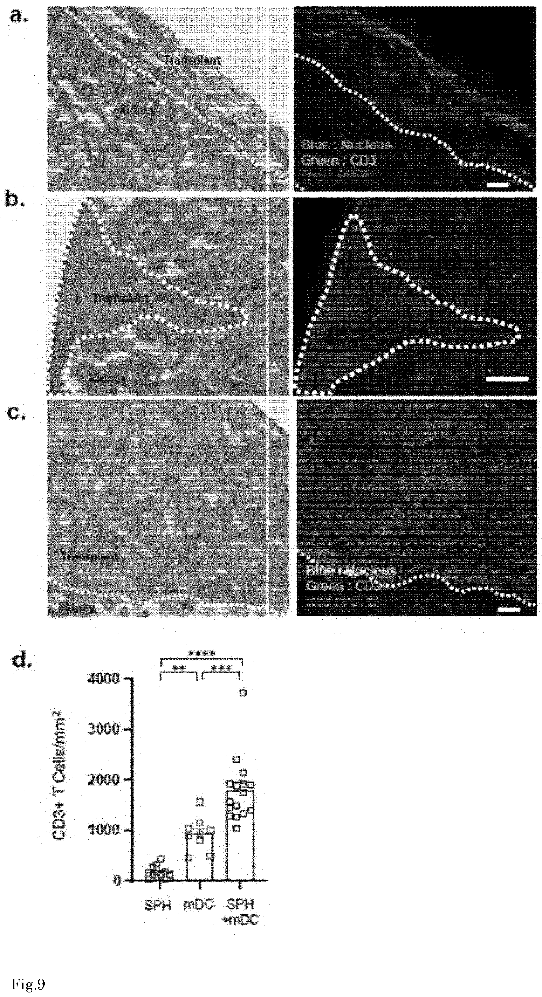

In order to check whether SVF spheroids have the ability to attract immune cells in vivo when SVF spheroids (SVF) were transplanted into the mouse kidney capsule, CD3+ T cells were not attracted, but when the mature dendritic cell (mDC) BMDCs were transplanted together, it was confirmed that many CD3+ T cells were attracted, and these CD3+ T cells were mixed with PDPN+ SVF cells (FIG. 9A: SVF spheroid alone transplanted group, 9B: mature dendritic cells alone transplanted group, 9C: transplant group injected with SVF spheroids and mature dendritic cells together). Even when only dendritic BMDCs were transplanted, CD3+ T cells were attracted, but it was confirmed that more CD3+ T cells were attracted when dendritic cells were transplanted with SVF spheroids (FIG. 9D).

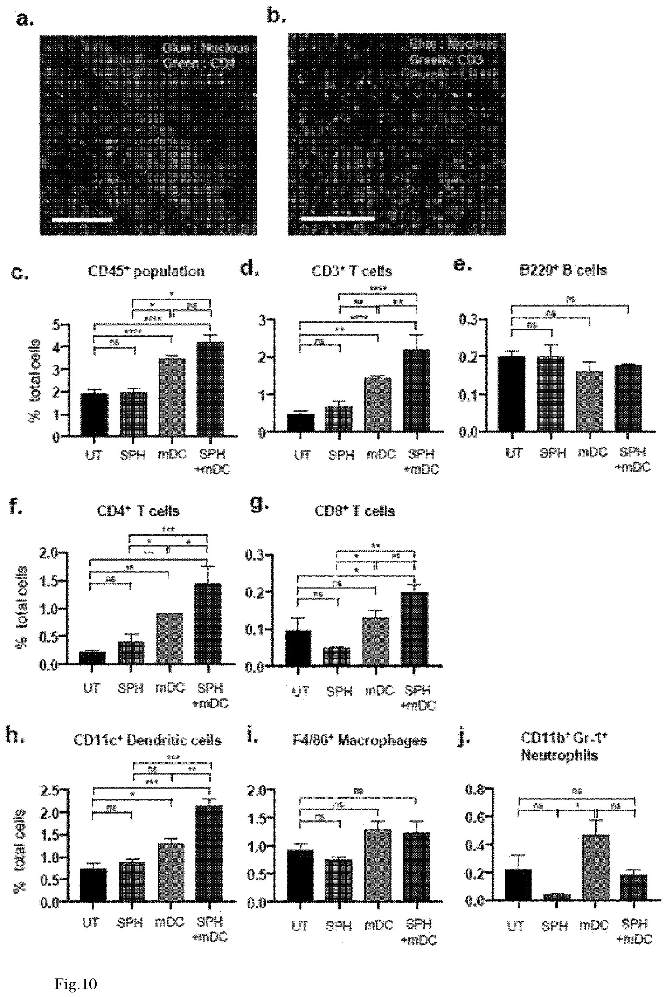

T cells attracted by the SVF spheroids contained CD4+, CD8+ T cells, and CD11c+ dendritic cells (FIGS. 10A and 10B), and when quantified by flow cytometry, it was confirmed that CD3+ T cells and CD4+ T cells were the most in the group transplanted with mature dendritic cells (mDC) and SVF spheroids (SPH+mDC), and a large number of CD8+ T cells and CD11c+ dendritic cells were also present (FIGS. 10C to 10J). These results can be said to show that SVF spheroids can increase CD3+ T cell infiltration when coexisting with dendritic cells.

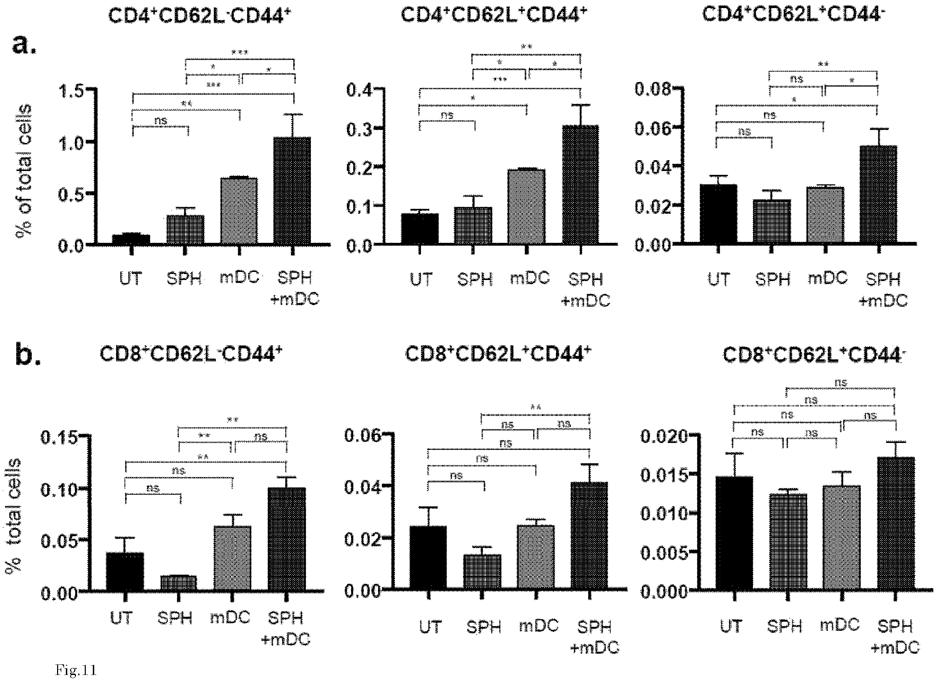

In the case that SVF spheroids are present together with dendritic cells when analyzed in more detail by flow cytometry to confirm the state of the induced T cells among CD62L-CD44+ effector T cells, CD62L+ CD44+ memory T cells, and CD62L+ CD44-immature T cells, CD4+ effective group T cells account for the largest portion among the T cells collected in the graft (FIGS. 11A and 11B).

The above results show that SVF spheroids attract dendritic cells and enhance dendritic cell interaction with T cells and that T cells are mostly differentiated into CD4+ effector T cells to perform an immune response.

2.4 The complex of SVF spheroids and dendritic cells enhances the response of antigen-specific T cells.

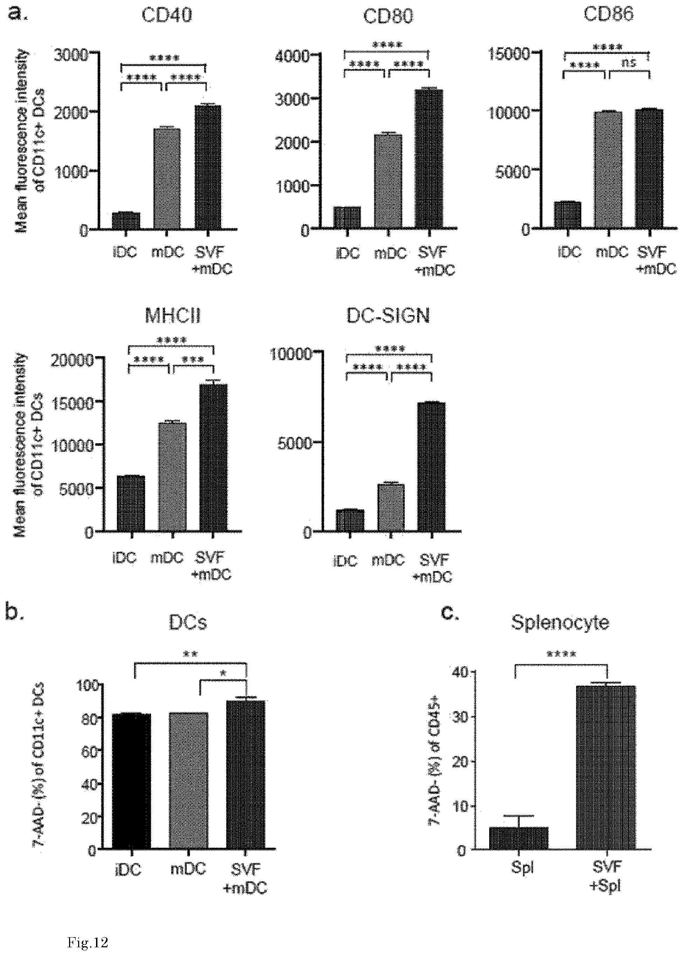

When SVF (two-dimensional culture of SVF) and mature dendritic cells (mature DC, mDC) were co-cultured to determine the mechanism by which SVF (two-dimensional culture of SVF) regulates the interaction between dendritic cells and T cells, it was confirmed that the expression of CD40, CD80, MHCII, and DC-SIGN, which are activation markers of dendritic cells, actually increased compared to immature dendritic cells (immature DC, iDC) and mature dendritic cells alone (FIG. 12A). In addition, when the two-dimensional culture SVF and mature dendritic cells were co-cultured for 1 day and stained with 7 AAD to examine the degree of apoptosis, it was confirmed that the survival rate of mature dendritic cells (SVF+mDC) was improved when co-cultured with SVF compared to the culture of immature dendritic cells (iDC) and mature dendritic cells (mDC) alone (FIG. 12B). Also, when SVF and ss, a two-dimensional culture, were co-cultured for 7 days and stained with 7 AAD, the survival rate of splenocytes (SVF+ Spl) co-cultured with SVF for 7 days was about 8 times higher than that of splenocytes alone (Spl) (FIG. 12C).

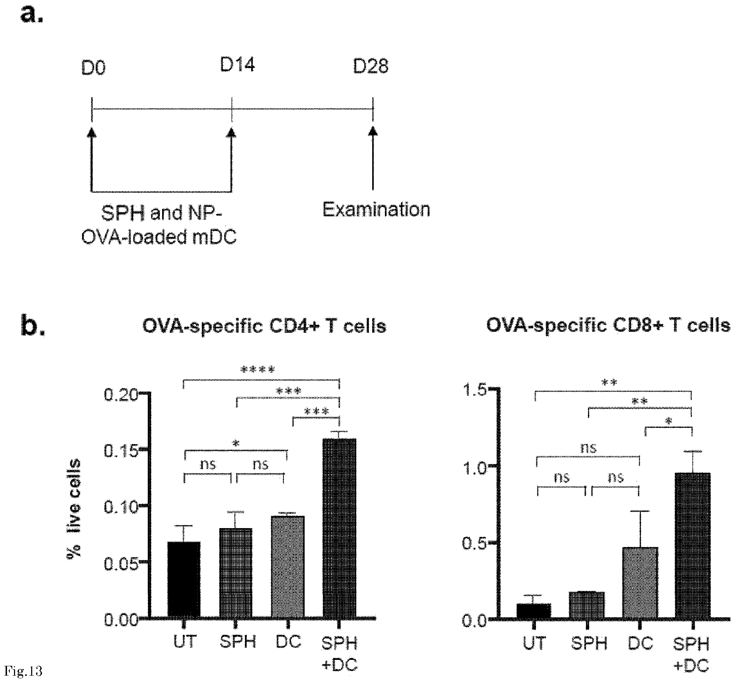

In order to examine whether the SVF spheroid and mDC complex can enhance the antigen-specific T cell response, the SVF spheroid and mDC complex was divided into (i) untreated (UT), (ii) SVF spheroid and OVA (ovabulbumin) transplanted group (SPH), (iii) a mature dendritic cell (mDC) transplanted group (mDC) loaded with OVA, (iv) a mature dendritic cell (mDC) transplanted group (SPH+ mDC) loaded with SVF spheroids and OVA (n = 5), and transplanted twice at the start of the experiment and at the second week of the experiment in the mouse kidney capsule (FIG. 13A). After 4 weeks, when the ratio of OVA antigen-specific CD4+, CD8+ T cells was examined by staining the OVA peptide presented in the MHC tetramer, it was confirmed that the ratio of CD4+, CD8+ T cells increased high in the mDC transplant group activated with SVF spheroids and OVA (FIG. 13B). Here, the antigen, OVA, was bound to Fe₃O₄-ZnO nanoparticles and used for loading into dendritic cells.

These results show that when mature dendritic cells are co-cultured with two-dimensional culture SVF or spheroids thereof or co-transplanted, the activation of dendritic cells is promoted, and the survival rate is improved, resulting in more effective activation of T cells and antigen-specific T cells, thereby enhancing the response of antigen-specific T cells.

### 2.5 Anti-tumor activity of SVF spheroid and dendritic cell complex

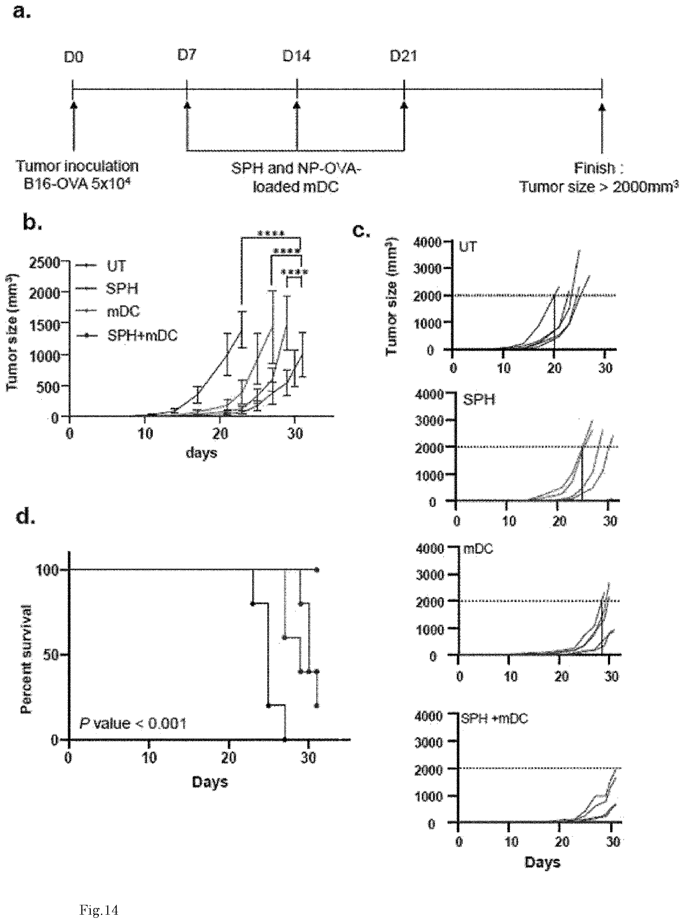

In order to confirm that the increased antigen-specific T cell immune response confirmed as above actually has an anti-cancer effect that inhibits the growth of cancer cells, B16 melanoma cells expressing OVA (B16-OVA) were injected in the flank of the mouse. The SVF spheroid and mDC complex was divided into (i) untreated (UT), (ii) SVF spheroid and OVA (ovalbumin) transplanted group (SPH), (iii) a mature dendritic cell (mDC) transplanted group (mDC) loaded with OVA, (iv) a mature dendritic cell (mDC) transplanted group (SPH+ mDC) loaded with SVF spheroids and OVA (n = 5) and transplanted to a mouse kidney capsule three times at 1-week intervals (FIG. 14A). Also here, the antigen, OVA, was bound to Fe₃O₄-ZnO nanoparticles and used for loading into dendritic cells. As a result of the experiment for 32 days, tumor growth was inhibited the most in the mDC transplanted group activated by SVF spheroids and OVA, and the survival rate was maintained at 100% during the experiment period (FIGS. 14B, 14C, and 14D). Therefore, it was found that the effect of SVF spheroids and dendritic cells increased compared to the injection of dendritic cells alone.

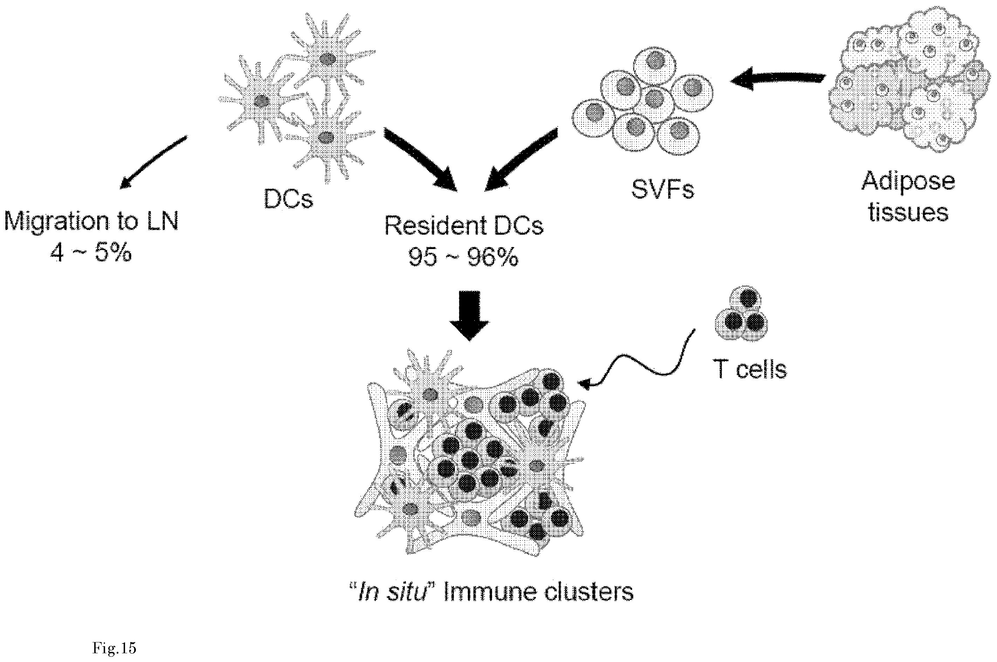

As described above, when the stromal vascular fraction (SVF) isolated from adipose tissue is transplanted together with dendritic cells at the dendritic cell transplantation site, SVF activates dendritic cells at the transplanted site and consequently enhances the response of T cells (FIG. 15).

## Claims

1. A composition for enhancing an immune response, the composition comprising a stromal vascular fraction isolated from adipose tissue and dendritic cells loaded with an antigen as active ingredients.

2. The composition of claim 1, wherein the stromal vascular fraction is a spheroid that is a two-dimensional culture or a three-dimensional culture.

3. The composition of claim 1, wherein the immune response is performed by T cells.

4. The composition of claim 1, wherein the antigen is a virus-derived antigen, a pathogenic microorganism-derived antigen, or a tumor-derived antigen.

5. The composition of claim 1, wherein the composition is an anti-viral immuno-enhancing composition for treating or preventing viral infection, an anti-microbial immuno-enhancing composition for treating or preventing an infection caused by a pathogenic microorganism, or an anti-tumor immuno-enhancing composition for tumor treatment or prevention.

6. The composition of claim 1, wherein the dendritic cells are autologous dendritic cells obtained from an individual to which the composition is administered, allogeneic dendritic cells obtained from another individual, or heterogeneous dendritic cells obtained from a heterogeneous individual.

7. The composition of claim 1, wherein the dendritic cells are immature dendritic cells or mature dendritic cells.

8. The composition of claim 1, wherein the adipose tissue is a subcutaneous fat tissue or a visceral fat tissue around an organ.

9. The composition of claim 1, wherein the antigen is a tumor-derived antigen, and the tumor-derived antigen is a surface-expressed protein of a tumor cell, a surface-expressed receptor of a tumor cell, a peptide inside a tumor cell, a protein inside a tumor cell, or a tumor cell lysate.

10. The composition of claim 1, wherein the antigen is a tumor-derived antigen, and the tumor-derived antigen is EGFRvIII, EGFR, metastin receptor, receptor tyrosine kinases, human epidermal growth factor receptor 2 (HER2), tyrosine kinase-18-receptor (c-Kit), HGF receptor c-Met, CXCR4, CCR7, endothelin-A receptor, peroxisome proliferator activated receptor δ (PPAR-δ), platelet-derived growth factor receptor α (PDGFR-α), CD133, carcinoembryonic antigen (CEA), epithelial cell adhesion molecule (EpCAM), disialoganglioside (GD2), glypican 3 (GPC3), prostate specific membrane antigen (PSMA), tumor-associated glycoprotein 72 (TAG-72), disialoganglioside (GD3), human leukocyte antigen-DR (HLA-DR), mucin 1 (MUC1), New York esophageal squamous cell carcinoma 1 (NY-ESO-1), latent membrane protein 1 (LMP1), tumor necrosis factor-related apoptosis-inducing ligand receptor (TRAILR2), vascular endothelial growth factor receptor 2 (VEGFR2), hepatocyte growth factor receptor (HGFR), CD44, or CD166.

11. The composition of claim 1, wherein the composition is used in combination with or mixed with an anti-viral agent, an anti-microbial agent, or an anti-cancer agent.

12. The composition of claim 1 is a pharmaceutical composition.

## Patentansprüche

1. Zusammensetzung zur Verstärkung einer Immunantwort, wobei die Zusammensetzung eine aus Fettgewebe isolierte stromale vaskuläre Fraktion und mit einem Antigen beladene dendritische Zellen als aktive Bestandteile umfasst.

2. Zusammensetzung nach Anspruch 1, wobei die stromale vaskuläre Fraktion ein Sphäroid ist, das eine zweidimensionale Kultur oder eine dreidimensionale Kultur ist.

3. Zusammensetzung nach Anspruch 1, wobei die Immunantwort von T-Zellen durchgeführt wird.

4. Zusammensetzung nach Anspruch 1, wobei das Antigen ein von einem Virus abgeleitetes Antigen, ein von einem pathogenen Mikroorganismus abgeleitetes Antigen oder ein von einem Tumor abgeleitetes Antigen ist.

5. Zusammensetzung nach Anspruch 1, wobei die Zusammensetzung eine antivirale immunstärkende Zusammensetzung zur Behandlung oder Verhinderung einer Virusinfektion, eine antimikrobielle immunstärkende Zusammensetzung zur Behandlung oder Verhinderung einer durch einen pathogenen Mikroorganismus verursachten Infektion oder eine antitumorale immunstärkende Zusammensetzung zur Tumorbehandlung oder -verhinderung ist.

6. Zusammensetzung nach Anspruch 1, wobei die dendritischen Zellen autologe dendritische Zellen sind, erhalten von einem Individuum, dem die Zusammensetzung verabreicht wird, allogene dendritische Zellen, erhalten von einem anderen Individuum, oder heterogene dendritische Zellen, erhalten von einem heterogenen Individuum.

7. Zusammensetzung nach Anspruch 1, wobei die dendritischen Zellen unreife dendritische Zellen oder reife dendritische Zellen sind.

8. Zusammensetzung nach Anspruch 1, wobei das Fettgewebe ein subkutanes Fettgewebe oder ein viszerales Fettgewebe um ein Organ herum ist.

9. Zusammensetzung nach Anspruch 1, wobei das Antigen ein von einem Tumor abgeleitetes Antigen ist und das vom Tumor abgeleitete Antigen ein an der Oberfläche exprimiertes Protein einer Tumorzelle, ein an der Oberfläche exprimierter Rezeptor einer Tumorzelle, ein Peptid innerhalb einer Tumorzelle, ein Protein innerhalb einer Tumorzelle oder ein Tumorzelllysat ist.

10. Zusammensetzung nach Anspruch 1, wobei das Antigen ein von einem Tumor abgeleitetes Antigen ist und das vom Tumor abgeleitete Antigen EGFRvIII, EGFR, Metastinrezeptor, Rezeptortyrosinkinasen, humaner epidermaler Wachstumsfaktorrezeptor 2 (HER2), Tyrosinkinase-18-Rezeptor (c-Kit), HGF-Rezeptor c-Met, CXCR4, CCR7, Endothelin-A-Rezeptor, Peroxisom-Proliferator-aktivierter Rezeptor δ(PPAR-δ), Thrombozyten-Wachstumsfaktor-Rezeptor α (PDGFR-α), CD133, Carcinoembryonales Antigen (CEA), Epitheliales Zelladhäsionsmolekül (EpCAM), Disialogangliosid (GD2), Glypican 3 (GPC3), prostataspezifisches Membranantigen (PSMA), tumorassoziiertes Glykoprotein 72 (TAG-72), Disialogangliosid (GD3), humanes Leukozytenantigen-DR (HLA-DR), Mucin 1 (MUC1), New Yorker Ösophaguskarzinom 1 (NY-ESO-1), latentes Membranprotein 1 (LMP1), Tumor-Nekrose-Faktor-bezogener Apoptose-induzierender Ligand-Rezeptor (TRAILR2), vaskulärer endothelialer Wachstumsfaktor-Rezeptor 2 (VEGFR2), Hepatozyten-Wachstumsfaktor-Rezeptor (HGFR), CD44 oder CD166 ist.

11. Zusammensetzung nach Anspruch 1, wobei die Zusammensetzung in Kombination mit oder gemischt mit einem antiviralen Mittel, einem antimikrobiellen Mittel oder einem Antikrebsmittel verwendet wird.

12. Zusammensetzung nach Anspruch 1 ist eine pharmazeutische Zusammensetzung.

## Revendications

1. Une composition pour renforcer une réponse immunitaire, la composition comprenant une fraction vasculaire stromale isolée du tissu adipeux et des cellules dendritiques chargées d'un antigène en tant qu'ingrédients actifs.

2. La composition selon la revendication 1, dans laquelle la fraction vasculaire stromale est un sphéroïde qui est une culture bidimensionnelle ou une culture tridimensionnelle.

3. La composition selon la revendication 1, dans laquelle la réponse immunitaire est réalisée par des cellules T.

4. La composition selon la revendication 1, dans laquelle l'antigène est un antigène dérivé d'un virus, un antigène dérivé d'un micro-organisme pathogène ou un antigène dérivé d'une tumeur.

5. La composition selon la revendication 1, dans laquelle la composition est une composition antivirale renforçant le système immunitaire pour traiter ou prévenir une infection virale, une composition antimicrobienne renforçant le système immunitaire pour traiter ou prévenir une infection causée par un micro-organisme pathogène, ou une composition antitumorale renforçant le système immunitaire pour le traitement ou la prévention d'une tumeur.

6. La composition selon la revendication 1, dans laquelle les cellules dendritiques sont des cellules dendritiques autologues obtenues à partir d'un individu auquel la composition est administrée, des cellules dendritiques allogéniques obtenues à partir d'un autre individu, ou des cellules dendritiques hétérogènes obtenues à partir d'un individu hétérogène.

7. La composition selon la revendication 1, dans laquelle les cellules dendritiques sont des cellules dendritiques immatures ou des cellules dendritiques matures.

8. La composition selon la revendication 1, dans laquelle le tissu adipeux est un tissu adipeux sous-cutané ou un tissu adipeux viscéral autour d'un organe.

9. La composition selon la revendication 1, dans laquelle l'antigène est un antigène dérivé d'une tumeur, et l'antigène dérivé d'une tumeur est une protéine exprimée en surface d'une cellule tumorale, un récepteur exprimé en surface d'une cellule tumorale, un peptide à l'intérieur d'une cellule tumorale, une protéine à l'intérieur d'une cellule tumorale, ou un lysat de cellules tumorales.

10. La composition selon la revendication 1, dans laquelle l'antigène est un antigène dérivé d'une tumeur, et l'antigène dérivé d'une tumeur est EGFRvIII, EGFR, récepteur de la métastine, récepteur tyrosine kinases, récepteur du facteur de croissance épidermique humain 2 (HER2), récepteur tyrosine kinase18 (c-Kit), récepteur HGF c-Met, CXCR4, CCR7, récepteur de l'endothéline-A, récepteur activé par le proliférateur de peroxisome δ (PPAR-δ), récepteur du facteur de croissance dérivé des plaquettes α (PDGFR-α), CD133, antigène carcinoembryonnaire (CEA), molécule d'adhésion des cellules épithéliales (EpCAM), disialoganglioside (GD2), glypican 3 (GPC3), antigène membranaire spécifique de la prostate (PSMA), glycoprotéine 72 associée à la tumeur (TAG-72), disialoganglioside (GD3), antigène leucocytaire humain-DR (HLA-DR), mucine 1 (MUC1), carcinome épidermoïde de l'œsophage de New York 1 (NY-ESO-1), protéine membranaire latente 1 (LMP1), récepteur du ligand inducteur d'apoptose lié au facteur de nécrose tumorale (TRAILR2), récepteur 2 du facteur de croissance endothélial vasculaire (VEGFR2), récepteur du facteur de croissance des hépatocytes (HGFR), CD44 ou CD166.

11. La composition selon la revendication 1, dans laquelle la composition est utilisée en combinaison avec ou mélangée avec un agent antiviral, un agent anti-microbien ou un agent anti-cancéreux.

12. La composition de la revendication 1 est une composition pharmaceutique.
